# EUROPEAN PATENT SPECIFICATION

(11) **EP 1 961 379 B1**
(45) Date of publication and mention of the grant of the patent: **04.09.2013**
(21) Application number: 08250301.2
(22) Date of filing: 24.01.2008
(51) Int. Cl.: A61B 5/107, A61B 19/00

(54) **Apparatus for indicating the bone thickness between a cavity in a bone and the bone surface**
Vorrichtung zur Bestimmung der Knochendicke zwischen einem Hohlraum in einem Knochen und der Knochenoberfläche
Appareils pour indiquer l'épaisseur de l'os entre une cavité dans un os et la surface de l'os

(30) Priority: 26.02.2007 GB 0703692
(43) Date of publication of application: 27.08.2008
(73) Proprietor: Stryker Ireland Limited, Carrigtwohill, Cork (IE)
(72) Inventor: Pinot, Loïc, 14400 Bayeux (FR); Rushton, Neil, Cambridgeshire, CB2 8AW (GB); Moindreau, Marie, 14000 Caen (FR); Field, Richard Eddy, Surrey, KT20 7UB (GB)
(74) Representative: Bridge-Butler, Jeremy

(56) References cited:
- EP-A- 1 563 795
- WO-A-03/034922
- US-A- 5 720 752
- US-B1- 6 602 258

## Description

This invention relates to apparatus for indicating the bone thickness between the predetermined distal end of a bone cavity located on a proximal/distal axis and the outer surface of the bone.

When preparing cavities in a bone, for example to receive the insert portion of a prosthetic component, it is necessary to drill into the bone. When fitting a prosthetic component to the proximal end of a femur when carrying out proximal epiphyseal replacement technique surgery, two proximal axially extending holes are drilled, one at an angle to the other, which can subsequently be joined by reaming to present a V-shaped cavity. When preparing such a cavity it is essential that one or other of the drill holes does not approach too close, or even break through, the wall of the bone. It is also common to prepare cavities, again, for example, at the end of a femur which are wide and if not accurately dimensioned, for example with regard to the width or the depth, can be too close to the wall of the bone, especially in the neck of the femur.

The present invention is intended to provide apparatus which will assist in determining the thickness of the bone between the distal end of a bone cavity and the outer surface of the bone.

The present invention provides an apparatus as defined by claim 1.

With this apparatus and knowing the accurate dimensions of the apparatus itself it is possible to measure the bone thickness concerned.

The apparatus may include a proximal/distal bone axis indicator which can, for example, be in the form of a guide wire. With this in place the guide wire can provide a datum which can be used to set up the apparatus.

In a preferred form the guide wire is provided by a guide pin which forms part of the means for location of the support member on the bone. Thus, the apparatus can be located on this accurately placed pin.

In an alternative arrangement the means for location of the support member on the bone may comprise a collar adapted to fit on the bone at a predetermined location.

The apparatus can also include extension means which can be adjusted to vary the proximal/distal length of the support member and which in one embodiment may comprise an adjustable carrier on which the adjustable distance indicating means is supported.

In an alternative construction the extension means may comprise a series of slots provided on said support member and adapted to locate on co-operating connection means carried on the means for location on the bone, use of alternate slots effectively increasing or decreasing the effective proximal/distal length of the support member.

In a preferred embodiment the adjustable distance indicating means include a sliding pin provided with distance indicating indicea which can be adjusted in relation to the support member.

The invention is not limited to operations relating to a femur and it can be used on any bone in which the distance between an opening of the wall is required.

The invention can be performed in various ways and two embodiments will now be described by way of example and with reference to the accompanying drawings in which :
Figure 1 is a diagrammatic side elevation of apparatus for indicating the bone thickness between the predetermined distal end of a bone cavity located on the proximal/distal axis and the outer surface of the bone according to the invention;
Figure 2 is a diagrammatic representation of the proximal end of a femur showing how it is prepared to carry out proximal epiphyseal replacement technique surgery;
Figure 3 is a diagrammatic cross-sectional representation of a prosthetic component for use in proximal epiphyseal replacement technique surgery and for which a prosthetic stem cavity can be prepared using the apparatus according to the invention;
Figure 4 is a diagrammatic cross-sectional view of an alternative form of apparatus according to the invention;
Figure 5 is an exploded isometric view of the apparatus shown in Figure 4;
Figure 6 is a side elevation of an alternative construction according to the invention;
Figure 7 is an exploded view of parts of the support member;
Figure 8 is an isometric view of the construction shown in Figure 6; and,
Figure 9 is a cross-sectional plan view on the line IX-IX of Figure 8.

The apparatus according to the invention is particularly, although not exclusively, for use with proximal epiphyseal replacement technique surgery. An example of this type of surgery is explained and shown in EP 1138 283 and includes resecting a femur at a position on the proximal side of its neck to locate a prosthetic femoral component which has a tapered insert portion and a proximal head portion. The insert portion is adapted for location in a prepared socket which, effectively, has inclined side to 5 provide a tapering opening.

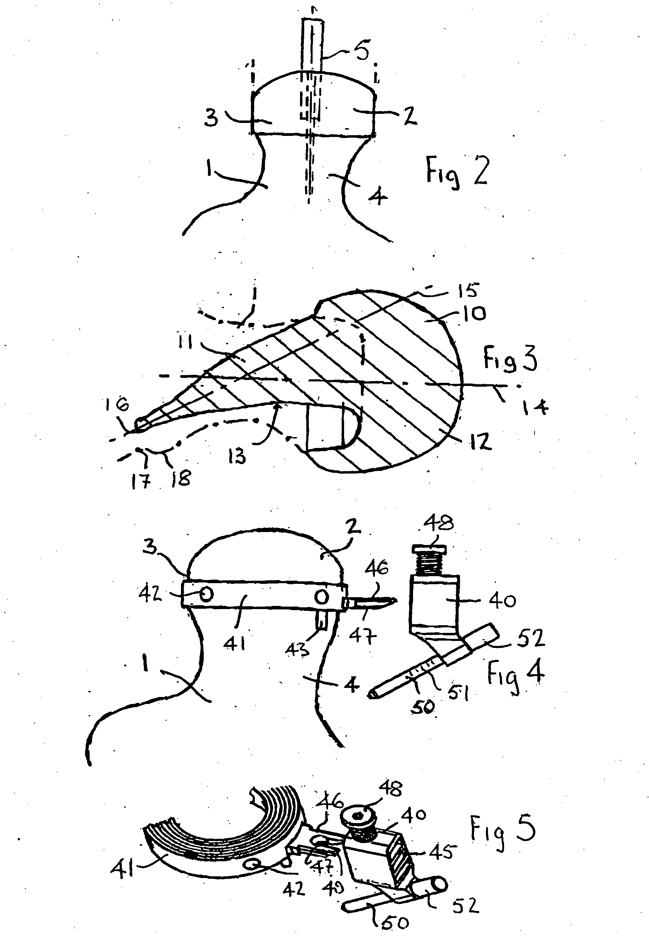

In order to carry out this type of surgery the end of a femur, indicated by reference numeral 1 in Figure 2, the femoral head, indicated by reference numeral 2, is first prepared by machining it with a cylindrical cutter (not shown) to provide a cylindrical portion 3. In order to accurately locate the cylindrical cutter a proximal/distal opening is drilled in the head to receive a guide wire and this opening is subsequently enlarged to receive a guide pin 5 on which the cylindrical cutter is located. This pin can also be used in the apparatus to be described herein.

The neck of the femur is indicated by reference numeral 4.

Figure 3 shows diagrammatically how a typical femoral head component for this type of surgery has a tapered insert portion 11 and a head 12 is fitted into a cavity 13 in the proximal end of the femur 1 which, in this Figure, is indicated by chain lines.

In order to provide the cavity 13 a hole is drilled into the prepared femur on a axis 14 which is substantially co-axial with the proximal/distal axis of the end of the femur. A second opening is also drilled along the line of another proximal/distal axis 15 which is an angle to the line 14 to provide the basis for the tapering socket.

By careful measurement of the femur and knowing the dimensions of the prosthetic component it is possible to accurately determine the position of the tip 16 of the component in the bone and the present apparatus can be used for indicating the bone thickness between the predetermined distal end of the bone cavity which is located on aproximal/distal axis and the outer surface of the bone, indicated by reference numeral 17. Reference numeral 18 indicates the calcar which can be used as a reference point.

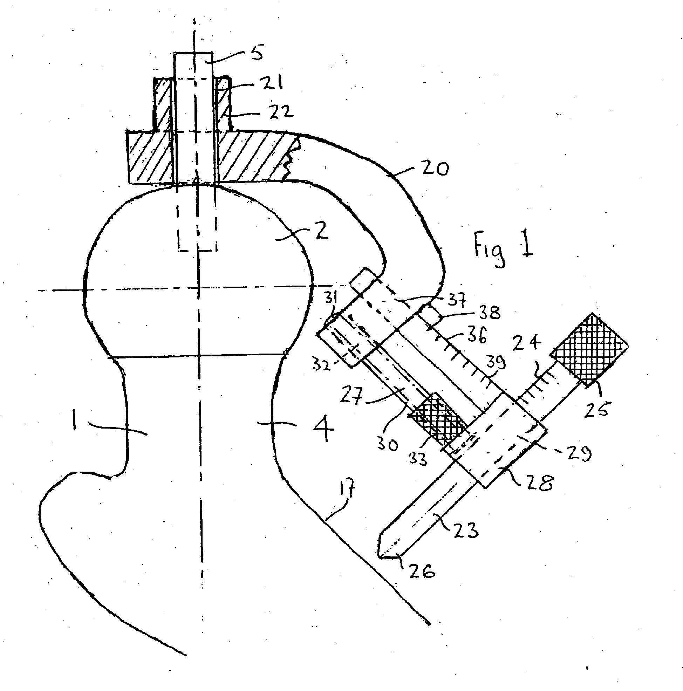

Apparatus according to the invention is shown in Figure 1 and the same reference numerals are used for similar parts, as indicated in Figure 2. In this Figure however the apparatus is being employed before the cylindrical cutter (not shown) is used to provide a cylindrical portion 3 and the guide pin 5 is used in connection with the present apparatus.

Apparatus according to the invention comprises a support member 20 provided with means for location on the bone in the form of a prepared socket 21 provided in a boss 22 which is dimensioned to be a close but sliding fit on the guide pin 5 which acts as a proximal/distal axis indicator. Thus the guide pin which was established on the guide wire provides a datum for setting up the apparatus. The support member 20 has an adjustable distance indicating means in the form of a sliding pin 23 which is provided with distance indicating indicea 24. The sliding pin 23 can be adjusted in relation to the support member 20 and carries an operating knob 25 for this purpose. As will be seen from Figure 1 the distal end 26 of the sliding pin 23 can be pushed into engagement with the surface 17 of the bone.

The apparatus also includes extension means 27 which can be adjusted to vary the proximal/distal length of the support member 20. These extension means comprise an adjustable carrier 28 on which the sliding pin 23 is supported in a bore 29. The carrier 28 is connected to the C-shaped support member 20 by a screw threaded shaft 30. The proximal end 31 of the screw-threaded shaft 30 is rotatable in a screw-threaded bore 32 and the distal end of the shaft 30 is located in the carrier 28 but is free to rotate. The shaft has an enlarged knurled portion 33 to assist in rotating it.

Location of the shaft 30 causes it to progress through the screw-threaded opening 32 so that the position of the carrier 28 relative to the support member 20 can be adjusted.

The carrier 28 also has a location strut 36 which is rigidly secured thereto and extends from the carrier through an opening 37 in the support member 20. An indicator fin 38 is provided on the support member 20 which aligns with indicea 39 provided on the location strut 36. With this arrangement therefore the carrier 28 accurately located on the support member 20 so that its proximal/distal position can be adjusted.

With this arrangement therefore, knowing the precise dimensions of the apparatus, it can be used to measure the distance from the predetermined position of a hole to be drilled in the head to measure the thickness of bone at the predetermined position.

In most circumstances this apparatus will merely confirm that the thickness of bone is adequate but if it proves to be too small then it will be known to the surgeon that he cannot use the normal surgical technique to drill the holes and it may be necessary to use some different technique for applying the prosthetic insert. Thus the apparatus can prove valuable in overcoming the difficulty of knowing whether a particular technique can be used or not. If, for example, proximal epiphyseal replacement technique surgery was intended and it was found that there was insufficient bone a different technique could be used whilst the bone was still undrilled. Initial drilling without previously measuring the bone thickness can do considerable damage to the head of the bone which could cause further difficulties if an alternative technique was subsequently to be employed.

Figures 4 and 5 show an alternative construction and in which similar reference numerals are used to indicate similar parts of the bone. This apparatus can conveniently be used on a head which has been prepared as shown in Figure 2 but in this case the pin 5 need not be employed because the support member 40 is located on the bone by a collar 41. The collar can be held in place by screws (not shown) being passed through screw-threaded holes 42. The collar also has a downwardly projecting pointer 43 which can be used to align the collar 41 in the desired position.

In this construction the support member 40 has a series of three slots 45 (as shown in Figure 5) which are adapted to be a close sliding fit on co-operating connecting means 46 which are in the form of a projecting lug 47 which fits into the slots 45. A spring loaded locking pin 48 is provided which can engage an enlarged opening 49 in the lug 47to rigidly hold the support member 40 in position. As the slots 45 are arranged in a proximal/distal direction alternative use of the slots can effectively extend or contract the length of the support member 40 in relation to the collar 41.

The distal end of the support member 40 carries a sliding pin 50 which is provided with indicea 51 and has an operating knob 52. This sliding pin 50 operates in a similar manner to the sliding pin 23 in the construction shown in Figure 1.

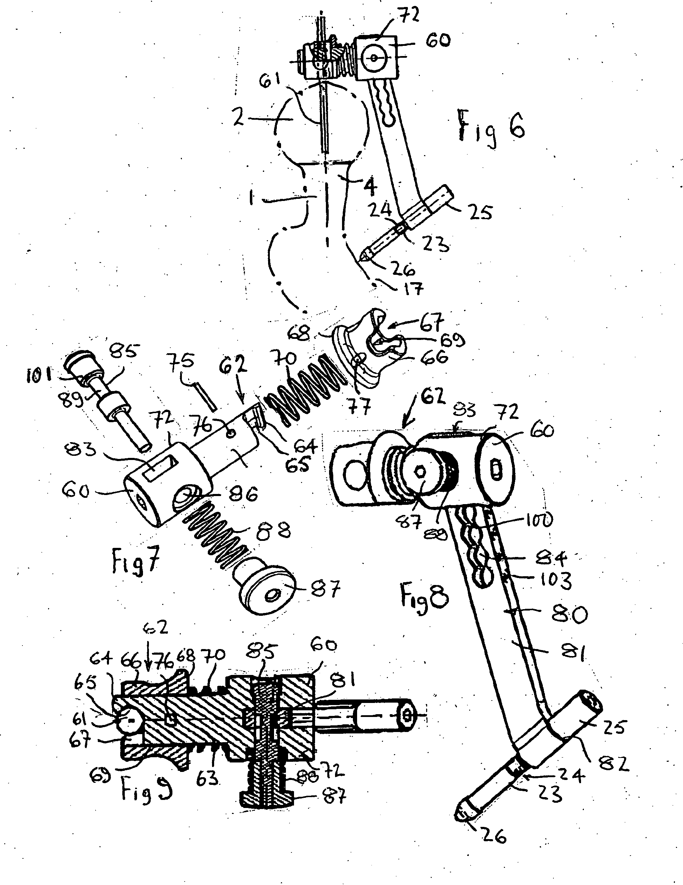

Figures 6, 7, 8 and 9 show another alternative construction which can be used in place of that shown in Figure 1. In Figure 6 the femur is indicated by chain dot lines and the same reference numerals are used to indicate similar parts as those shown in Figure 1. The apparatus comprise a support member 60 provided with means for location on the bone in the form of a clamp 62 which is designed to engage and clamp on a guide wire 61.

The clamp 62 comprises a cylindrical portion 63 provided on the support member 60 and the distal end of which is provided with a claw-shaped portion 64 which has an inner curved surface 65 shaped to engage the guide wire 61. The guide wire is held in place by a clamping sleeve 66 which is bifurcated to forma shaped slot 67 at its proximal end and a flat flange 68 at its distal end.

The bore 69 of the clamping sleeve 66 is dimensioned to be a sliding fit on the cylindrical portion 63.

From Figure 7 it will be seen that the clamping sleeve 66 is assembled over the cylindrical portion 63 with a compression coil spring 70 which also fits over the cylindrical portion 63 and is located between the flange 68 on the clamping sleeve 66 and the end of an enlarged portion 72 of the support member 60. In order to retain the clamping sleeve 66 in place a pin 75 is provided which is located in a hole 76 in the cylindrical portion 63. The pin 75 is long enough to project from one side of the hole 76 and engage at that end in a slot 77 which extends through the wall of the clamping sleeve. As the slot 77 is elongated it acts to hold the sleeve 66 in place but allows sliding movement for the length of the slot between the parts.

Figure 9 shows how the shaped slot 67 in the clamping sleeve 66 extends around the claw 62 and, when a guide wire 61 is in place, acts to clamp it against the inner curved surface 65 the claw 62. To release the wire 61 it is merely necessary to move the clamping sleeves 66 against the action 62 of the spring 70 along the cylindrical portion 63 so that the wire 61 can be withdrawn from the inner curved surface 65 of the claw 64. Thus the guide wire 61 provides a datum for setting up the apparatus.

The support member 60 has an adjustable distance indicating means similar to that shown in Figure 1 in that it includes a sliding pin 23 which is provided with distance indicating indicea 24. The sliding pin 23 can be adjusted in relation to the support member 60 and carries an operating knob 25 for this purpose. As will be seen from Figure 6 the distal end 26 of the sliding pin 23 can be pushed into engagement with the surface 17 of the bone.

The adjustable distance indicating means is connected to the support member 60 by extension means 80 which can be adjusted to vary the proximal/distal length of the support member 60. These extension means comprise an adjustable carrier 81 on which the sliding pin 23 is supported in a bore 82. The carrier 81 is connected to the support member 60 by passing through a slot 83, best shown in Figure 7. The carrier means 81 is in the form of a flat bar 81 and is provided with a series of linked openings 84. The bar 81 can be locked in a number of proximal/distal positions by operation of a locking pin 85 which is assembled in a bore 86 in the enlarged portion 72 of the support member 60. As will be seen from Figure 9 the pin passes through the bore 86 and extends on the far side where it is engaged by an operating button 87. The button is screw threaded onto the end of the locking pin 85 and a compression spring 88 is located between the operating button 87 and the end of the bore 86 so that the button can be moved towards the bore thus causing the pin 85 to move with it and allow a waisted portion 89 of the pin to be aligned with the slot 72. The dimensions of the waisted portion 89 of the pin are slightly less than the gaps 100 between the openings 84 in the flat bar 81 and this enables the bar to be moved lengthwise within the slot 72 to vary its operative length. The selected length can be clamped into position by releasing the operating button 86 to allow the locking pin 85 to move into its innermost position, as shown in Figure 9, where an enlarged portion 101 of the locking pin 85 is engaged in one of the openings 84 of the bar 81 and thus holding it in position.

Suitable indicea 103 is provided on the edge of the extension so that its precise length can be ascertained by the operator.

The apparatus is operated in the same way as that described with regard to the apparatus shown in Figure 1.

The apparatus is not exclusively for use when carrying out proximal epiphyseal replacement technique surgery but can be used in many other operations in which a hole has to be drilled into a bone and where it is desirable to know the bone thickness between the end of the hole and the surface of the bone.

## Claims

1. Apparatus for indicating the bone thickness between the predetermined distal end of a bone cavity (13) located on a proximal/distal axis of a bone (1) and the outer surface of the bone (1) comprising a support member (20) provided with means (5,21) for fixedly attaching it on the outer surface of the bone and in a predetermined position and said support member (20) having adjustable distance indicating means (23) which is adapted to be adjusted in relation to said support member (20) to contact the outer surface (17) of the bone (1), **characterised in that** said adjustable distance indicating means (23) is adapted to indicate a distance between the predetermined distal end of the bone cavity (13) and the outer surface (17) of the bone (1).

2. Apparatus as claimed in claim 1 which includes a proximal/distal bone axis indicator (5).

3. Apparatus as claimed in claim 2 in which said proximal/distal bone axis indicator is provided by a guide wire.

4. Apparatus as claimed in claim 3 in which said guide wire is provided by a guide pin (5) which forms part of the means (21) for location of the support member (20) on the bone (1).

5. Apparatus as claimed in claim 1 in which the means (21) for location of the support member (20) on the bone (1) comprises a collar (41) adapted to fit on the bone (1) at a predetermined location.

6. Apparatus as claimed in claims 1 to 5 including extension means (27) which can be adjusted to vary the proximal/distal length of the support member (20).

7. Apparatus as claimed in claim 6 in which the extension means (27) comprises an adjustable carrier (28) on which the adjustable distance indicating means (23) is supported.

8. Apparatus as claimed in claim 6 in which the extension means (27) comprises a series of clots (45) provided in said adjustable carrier (28) adapted to locate on co-operating connection means (46) carried on said means (21) for location on the bone.

9. Apparatus as claimed in any one of the preceding claims 1 to 8 in which the adjustable distance indicating means (23) include a sliding pin (23) provided with distance indicating indicea (24) which is adapted to be adjusted in relation to the support member (20).

## Patentansprüche

1. Vorrichtung zum Anzeigen der Knochendicke zwischen dem auf einer proximal/distalen Achse eines Knochens gelegenen vorbestimmten distalen Ende einer Knochenhöhle (13) und der äußeren Oberfläche des Knochens (1), umfassend ein Stützglied (20), das mit Mitteln (5,21) versehen ist, um es fest auf der äußeren Oberfläche des Knochens und in einer vorbestimmten Position zu befestigen, und wobei das Stützglied (20) ein verstellbares Entfernungsanzeigemittel (23) aufweist, das angepasst ist, um es in Bezug zu dem Stützglied (20) zu verstellen, um mit der äußeren Oberfläche (17) des Knochens (1) in Berührung zu treten, **dadurch gekennzeichnet, dass** das verstellbare Entfernungsanzeigemittel (23) angepasst ist, um eine Entfernung zwischen dem vorbestimmten distalen Ende der Knochenhöhle (13) und der äußeren Oberfläche (17) des Knochens (1) anzuzeigen.

2. Vorrichtung nach Anspruch 1, welche einen proximal/distale Knochenachse-Anzeiger (5) einschließt.

3. Vorrichtung nach Anspruch 2, bei welcher der proximal/distale Knochenachse-Anzeiger (5) durch einen Führungsdraht bereitgestellt wird.

4. Vorrichtung nach Anspruch 3, bei welcher der Führungsdraht durch einen Führungsstift (5) bereitgestellt wird, der einen Teil der Mittel (21) zur Positionierung des Stützgliedes (20) auf dem Knochen (1) bereitgestellt wird.

5. Vorrichtung nach Anspruch 1, bei welcher das Mittel (21) zur Positionierung des Stützgliedes (20) auf dem Knochen (21) einen Kragen umfassen, der angepasst ist, um an einer vorbestimmten Stelle auf den Knochen (1) zu passen.

6. Vorrichtung nach den Ansprüchen 1 bis 5, einschließend ein Verlängerungsmittel (27), das verstellt werden kann, um die proximal/distale Länge des Stützgliedes (20) zu verändern.

7. Vorrichtung nach Anspruch 6, bei welcher das Verlängerungsmittel (27) einen verstellbaren Träger (28) umfasst, auf dem das verstellbare Entfernungsanzeigemittel (23) abgestützt ist.

8. Vorrichtung nach Anspruch 6, bei welcher das Verlängerungsmittel (27) eine Reihe von Schlitzen (45) umfasst, die in dem verstellbaren Träger (28) vorgesehen sind, wobei sie angepasst sind, um sie auf zusammenwirkenden Verbindungsmitteln (46) zu positionieren, die auf den Mitteln (21) zur Positionierung auf dem Knochen getragen werden.

9. Vorrichtung nach einem der vorangehenden Ansprüche 1 bis 8, bei welcher die verstellbaren Entfernungsanzeigemittel (23) einen mit Entfernungsanzeigemarkierungen (24) versehenen Schiebestift (23) einschließen, der angepasst ist, um in Bezug zum Stützglied (20) verstellt zu werden.

## Revendications

1. Appareil pour indiquer l'épaisseur d'un os entre l'extrémité distale prédéterminée d'une cavité (13) de l'os située sur un axe proximo-distal d'un os (1) et la surface extérieure de l'os (1), comportant un élément de support (20) pourvu de moyens (5, 21) pour l'immobiliser sur la surface extérieure de l'os et dans une position prédéterminée et ledit élément de support (20) ayant un moyen réglable (23) d'indication de distance conçu pour être réglé par rapport audit élément de support (20) pour venir au contact de la surface extérieure (17) de l'os (1), **caractérisé en ce que** ledit moyen réglable (23) d'indication de distance est conçu pour indiquer une distance entre l'extrémité distale prédéterminée de la cavité (13) de l'os et la surface extérieure (17) de l'os (1).

2. Appareil selon la revendication 1, comportant un indicateur (5) d'axe proximo-distal d'os.

3. Appareil selon la revendication 2, dans lequel ledit indicateur d'axe proximo-distal d'os est constitué par un câble-guide.

4. Appareil selon la revendication 3, dans lequel ledit câble-guide est constitué par une tige de guidage (5) qui fait partie du moyen (21) pour la mise en place de l'élément de support (20) sur l'os (1).

5. Appareil selon la revendication 1, dans lequel le moyen (21) pour la mise en place de l'élément de support (20) sur l'os (1) comprend un collier (41) conçu pour s'ajuster sur l'os (1) à un endroit prédéterminé.

6. Appareil selon les revendications 1 à 5, comportant un moyen de déploiement (27) réglable pour modifier la longueur proximo-distale de l'élément de support (20).

7. Appareil selon la revendication 6, dans lequel le moyen de déploiement (27) comprend un support réglage (28) sur lequel repose le moyen réglable (23) d'indication de distance.

8. Appareil selon la revendication 6, dans lequel le moyen de déploiement (27) comprend une série de reliefs (45) présents dans ledit support réglable (28), destinés à se placer sur des moyens de montage (46), avec lesquels ils coopèrent, portés par ledit moyen (21) pour la mise en place sur l'os.

9. Appareil selon l'une quelconque des revendications précédentes 1 à 8, dans lequel le moyen réglable (23) d'indication de distance comprend une tige coulissante (23) pourvue de repères (24) d'indication de distance, qui est conçue pour être réglée par rapport à l'élément de support (20).
